(19) Europäisches Patentamt
European Patent Office
Office européen des brevets

(11) Numéro de publication: **0 191 675**
**B1**

(12) **FASCICULE DE BREVET EUROPÉEN**

(45) Date de publication du fascicule du brevet: **14.12.88**

(51) Int. Cl.⁴: **C 07 C 6/04,** B 01 J 31/02

(21) Numéro de dépôt: **86400140.9**

(22) Date de dépôt: **24.01.86**

(54) **Perfectionnement à la métathèse d'oléfines avec un catalyseur à base d'un complexe de tungstène.**

(30) Priorité: **12.02.85 FR 8501930**

(43) Date de publication de la demande: **20.08.86 Bulletin 86/34**

(45) Mention de la délivrance du brevet: **14.12.88 Bulletin 88/50**

(84) Etats contractants désignés: **CH DE GB IT LI NL**

(56) Documents cité:
**EP-A-0 129 474**
**FR-A-2 525 224**

(73) Titulaire: **SOCIETE NATIONALE ELF AQUITAINE, Tour Aquitaine, F-92400 Courbevoie (FR)**

(72) Inventeur: **Basset, Jean Marie Maurice, 21 Petite rue de la Doua, F-69100 Villeurbanne (FR)**
Inventeur: **Leconte, Michel, 26 rue Jean- Claude Vivant, F-69100 Villeurbanne (FR)**
Inventeur: **Quignard, Françoise, 15 rue du Chariot d'Or, F-69004 Lyon (FR)**
Inventeur: **Ollivier, Jean, Croix de Buzy, F-64260 Arudy (FR)**

(74) Mandataire: **Kohn, Armand, 5 Avenue Foch, F-92380 Garches (FR)**

**EP 0 191 675 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

L'invention concerne un perfectionnement à la métathèse de différentes oléfines par l'application d'un catalyseur amélioré. Elle comprend, en tant que nouveau produit industriel, ce catalyseur particulier, constitué par un complexe organique du tungstène.

La métathèse, c'est-à-dire dismutation, d'oléfines a fait l'objet, ces temps derniers, de nombreuses études, à cause de l'intérêt pratique qu'elle présente. La demanderesse a réalisé un progrès important par le choix de catalyseurs plus intéressants, décrits dans les FR-A-2 547 513 et FR-A-2 565 505. Le premier de ces documents vise les catalyseurs du type

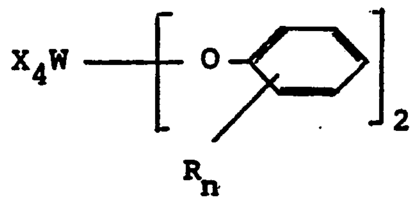

(1)

où X est un halogène, R un groupe hydrocarboné, groupe ou atome électronégatif, et n un nombre entier de 1 à 5. De tels catalyseurs associés à des composés de Al ou Sn ont permis de dismuter diverses oléfines bien plus rapidement que ne le font les catalyseurs de la technique antérieure. Selon le second des brevets cités ci-dessus, l'activité du nouveau catalyseur est encore augmentée par l'adjonction de composés de Pb au lieu de composés de Sn.

La présente invention apporte un perfectionnement par rapport à la technique antérieure, y compris celle des publications notées plus haut. En effet, l'utilisation d'un catalyseur suivant l'invention permet une métathèse aussi rapide, ou plus rapide, sans co-catalyseur, et cela aussi bien pour les oléfines portant des groupes fonctionnels, que pour celles qui n'en portent pas.

Cependant l'addition d'un co-catalyseur acide de Lewis, par exemple des composés organo métalliques à base d'Al, Sn, Pb, Mg ou Ti tels que décrits et connus dans la technique de la métathèse d'oléfine, et particulièrement dans les FR-A-2 547 513 et FR-A-2 565 505, conduit à des systèmes catalytiques encore plus actifs.

Un autre avantage de l'invention sur la technique connue réside en ce qu'elle rend possible l'utilisation de catalyseurs présentant à la fois une bonne activité et une stéréo-sélectivité on peut ainsi obtenir une excellente stéréo-sélectivité, jusqu'à des conversions proches des conversions maximales, correspondant à l'équilibre de la réaction de métathèse. Si l'on se réfère à ce qui est indiqué dans la littérature technique, notamment R.H. GRUBBS, "Comprehensive Organometallic Chemistry", 8 (54) (1982) p 499 - 551, J.M. BASSET, G. COUDURIER, R. MUTIN et H. PRALIAUD, "Journal of Catalysis", 34 (1974) p.152 - 155, J.L. BILHOU, J.M. BASSET, R. MUTIN et W.F. GRAYDON, "Journal of American Chemical Society", 99 (1977) p.4083 - 4090, M. LECONTE, Y. BEN TAARIT, J.L. BILHOU et J.M. BASSET, "Journal of Molecular Catalysis", 8 (1980) p. 263 - 268, très peu de catalyseurs de métathèse s'avèrent stéréosélectifs, même à faible conversion; d'autre part, il ressort également de cette littérature qu'avec la plupart des systèmes catalytiques classiques, lorsque la conversion de la réaction de métathèse augmente, il se produit parallèlement une isomérisation cis-trans; celle-ci entraîne, pour les produits de la réaction, une perte de la stéréosélectivité qui s'approche rapidement, avec l'avancement de la réaction, des valeurs données par l'équilibre thermodynamique.

Un cas de catalyseur stéréosélectif a été cité par T.J. KATZ et W.H. HERSH, "Tetrahedon Letters", n°6, (1977) p. 585 - 588. Cependant, comme l'indique l'article, ce catalyseur s'avère, d'une part, très peu actif et, d'autre part, stéréosélectif uniquement dans la métathèse de pen-tène-2-cis alors qu'il ne l'est pas dans la métathèse du pentène-2-trans. Le catalyseur de la présente invention apporte un progrès important, puisqu'il est beaucoup plus actif et qu'il peut réaliser de façon stéréosélective aussi bien la métathèse d'une oléfine cis que celle d'une oléfine trans.

Pour toutes ces raisons, en particulier du fait de leur stéréosélectivité, les catalyseurs de l'invention sont fort intéressants pour la synthèse des phéromones d'insectes. En effet, nombre de ces composés peuvent être obtenus par métathèse d'oléfines porteuses de groupes fonctionnels et ne sont souvent biologiquement actifs que sous une forme stéréochimique déterminée (cis ou trans).

Dans le catalyseur perfectionné suivant l'invention, constitué par un complexe diphénoxy du tungstène les autres groupements de coordination ou ligandes comprennent au moins un halogène et des groupes hydrocarbonés dont un est un carbène.

De façon générale, ce catalyseur peut être représenté par la formule

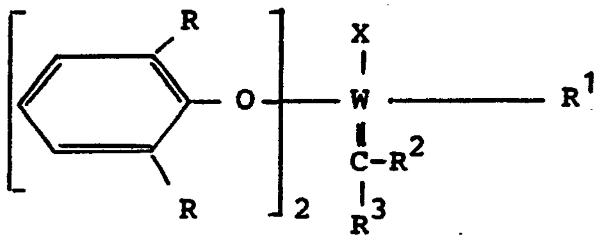

(2)

où X est un halogène, R un groupe hydrocarboné ou un groupe ou atome électronégatif, $R^1$ un groupe alkyle, de préférence en $C_1$ à $C_8$, linéaire ou ramifié et $R^2$ et $R^3$ des atomes d'hydrogène ou des groupes alkyles linéaires ou ramifiés en $C_1$ à $C_8$ ; $R^1$, $R^2$ et $R^3$ peuvent être semblables ou différents.

Des R préférés sont des alkyles en $C_1$ à $C_6$, des phényles ou des atomes de Cl, Br ou F.

Les groupes $R^1$, $R^2$ et $R^3$, semblables ou différents, comprennent par exemple les groupes linéaires ou ramifiés suivants :

$-CH_3$, $-(CH_2)_n-CH_3$

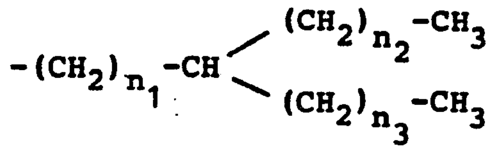

(n nombre entier allant de 1 à 7) ($n_1$, $n_2$, $n_3$ nimbres entiers égaux ou différents allant de 0 à 5 avec $0 \leqslant n_1 + n_2 - n_3 \leqslant 5$)

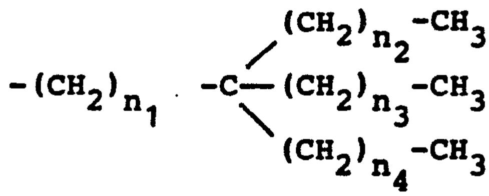

($n_1$, $n_2$, $n_3$, $n_4$ nombres entiers égaux ou différents allant de 0 à 4 avec $0 \leqslant n_1 + n_2 + n_3 + n_4 \leqslant 4$)

De plus, $R^2$ ou $R^3$ ou les deux peuvent être des atomes d'hydrogène. Enfin, $R^2$ et $R^3$ peuvent appartenir à la même chaîne hydrocarbonée comme par exemple : $= C\ (CH_2)_n$ dans laq uelle $4 \leqslant n \leqslant 10$.

Dans une forme préférée de l'invention, les groupes $R^1$, $R^2$ et $R^3$ sont tels que le groupe $R^1$ peut s'écrire sous la forme

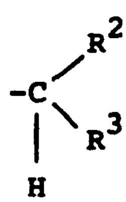

A titre d'exemple, dans une forme plus particulièrement préférée, $R^1$ est un groupe néo-pentyle

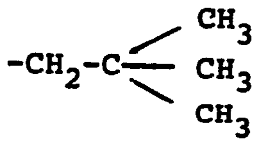

$R^2$ un groupe tertio-butyle

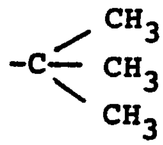

et $R^3$ un atome d'hydrogène.

Des complexes du tungstène possédant un ligande carbène, utilisables dans la réaction de métathèse des oléfines, ont été décrits dans le FR-A-2 525 224; toutefois ces catalyseurs font intervenir dans leur formule deux métaux différents et un composé acide de Lewis. Ils sont donc notoirement différents des catalyseurs de la présente invention, puisque ces derniers ne fônt intervenir qu'un seul métal et pas nécessairement un composé acide de Lewis.

On peut obtenir les catalyseurs de l'inventions en faisant réagir un complexe phénoxy de tungstène de formule (1) avec un composé organo-magnésien de formule générale $Mg(CHR'R'')_2$ (dioxanne) où R' et R'' sont des atomes d'hydrogène ou des groupes hydrocarbonés alkyles. Dans une forme d'exécution particulière, préférée, R' est un atome d'hydrogène et R'' un groupe tertiobutyle: $C(CH_3)_3$. Le rapport molaire organo-magnésien/phénoxy-tungstène utilisé dans la réaction est de préférence égal à 1,5. La réaction est conduite dans un solvant généralement éther qui peut être un éther acyclique ou cyclique. De préférence cet éther est tel que diméthyl-éther, diéthyl-éther, diisopropyl-éther, tétrahydrofurane ou dioxanne.

Au cours de la réaction, menée à une température qui peut être comprise entre -20°C et +35°C et de préférence à ou au voisinage de 250°C, il se forme rapidement un précipité de $MgX_2$ ou $MgX_2$ (dioxanne) qui est éliminé, et un deuxième produit qui reste en solution dans l'éther. La solution éthérée est récupérée et, après élimination de l'éther, on obtient le produit de la formule (2), qui constitue le catalyseur selon l'invention.

En général, une molécule de l'éther, qui a servi de solvant, reste combinée au complexe; cela peut être représenté par la formule 3:

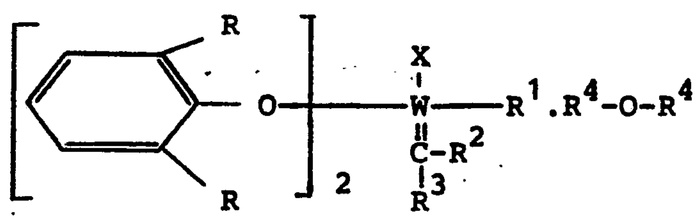 (3)

où $R^4$ est un alkyle en $C_2$ à $C_6$, normal ou ramifié, et de préférence en $C_2$ ou $C_3$, $R^4$-O-$R^4$ peut d'ailleurs se trouver sous une forme cyclique, notamment 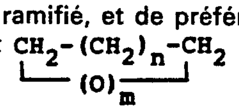

où n est 0 à 4 et m = 1 ou 2.

La molécule d'éther attachée au complexe n'est pas un co-catalyseur mais plutôt un ligande qui fait bien partie de la sphère de coordination du tungstène. La nature de ce ligande éther peut modifier la propriété du catalyseur au même titre, par exemple, que la nature des ligandes phénoxy. La formule du catalyseur pourrait d'ailleurs s'écrire ainsi:

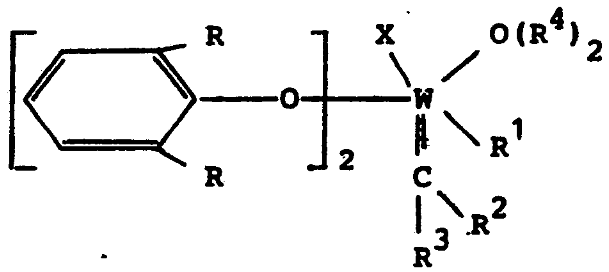

La métathèse, utilisant le nouveau catalyseur suivant l'invention, peut être réalisée à la manière connue en soi. Bien que la proportion d'oléfine traitée par mole de complexe tungstique puisse varier largement suivant l'activité du catalyseur, elle est le plus souvent de 30 à 300 moles par atome de W présent, et de préférence de 50 à 150 mols/W.

La température, également variable avec la nature des réactifs en présence, est en général de 0 à 100° C et principalement de 500 à 95° C.

Des catalyseurs suivant l'invention ont été essayés dans la métathèse de quelques oléfines en l'absence de tout co-catalyseur. Les exemples ci-après montrent les résultats intéressants obtenus.

EXEMPLE 1

Préparation d'un catalyseur perfectionné suivant l'invention (J)

Dans un tube de Schlenk, préalablement purgé à l'argon sec, on introduit 20 ml de diéthyl-éther anhydre, 0,093 g (1,15 x $10^{-4}$ mole, du complexe précurseur de formule :

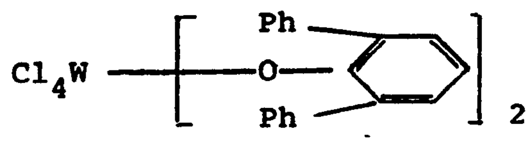

(4)

et 0,043 g (1,7 x $10^{-4}$ mole, du composé organo-magnésien $Mg(CH_2C(CH_3)_3)_2$ (dioxanne).

La solution éthérée prend instantanément une couleur brune, tandis que se forme un précipité blanc de $MgCl_2$ (dioxanne). Après environ 30 minutes de réaction à la température ambiante, la solution éthérée est décantée, filtrée et transférée dans un second tube de Schlenk sous argon. Après évaporation de l'éther, on récupère 0,085 g (rendement 80 %) d'une poudre de couleur ocre dont l'analyse élémentaire est conforme à la formule :

(J) ...

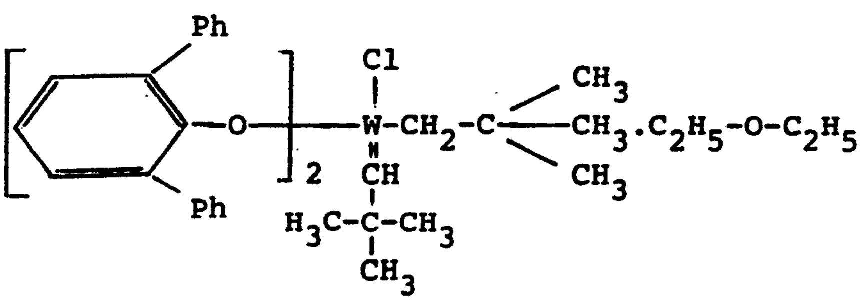

Analyse

calculé : C=64,9 % ; H=6,2 % ; Cl=3,8 % ; W=19,9 %
trouvé : C=63,6 % ; H=5,7 % ; Cl=3,9 % ; W=20,1 %

Le spectre de résonance magnétique nucléaire donne pour le proton H du ligande carbène $=CHC(CH_3)_3$, un signal de résonance ayant un déplacement chimique de: $\delta$ = 9,96 ppm/TMS. Le catalyseur, ainsi synthétisé, est désigné par (J) dans la suite de la présente description. De façon analogue ont été synthétisés des catalyseurs par réaction des précurseurs homologues à celui de la formule (4), dans lesquels Ph est remplacé par -Cl, -F, ou $CH_3$, avec le composé organo-magnésien Mg $(CH_2C(CH_3)_3)_2$ dioxanne.

EXEMPLES 2 à 6

Métathèse d'une oléfine non porteuse de groupes fonctionnels (pentène-2-cis)

Dans ces exemples sont comparées les activités et les stéréosélectivités de quatre catalyseurs suivant l'invention, D, E, J et K, avec celles du système catalytique classique $WCl_6 + Sn(CH_3)_4$.

Les catalyseurs D, E et K répondent à la même formule que J de l'exemple 1, sauf que dans:

D les Ph sont remplacés par des F,
E les Ph sont remplacés par des $CH_3$,
K les PH sont remplacés par des Cl

Les essais consistent à effectuer la métathèse du pentène-2-cis suivant la réaction:
$2CH_3CH = CHCH_2CH_3 \rightleftarrows CH_3CH=CHCH_3 + CH_3CH_2CH=CHCH_2CH_3$
donnant du butène-2 et de l'héxène-3.

Dans un réacteur pour travail discontinu, préalablement purgé à l'argon, on introduit d'abord $20x10^{-6}$ moles de composé tungsténique correspondant à D, E, J, K, et 100 x $106^{-6}$ dans le cas du $WCl_6 + Sn(CH_3)_4$; ensuite on y verse 5 ml de chlorobenzène servant de solvant. Le réacteur est chauffé à 85°C et le pentène-2-cis est introduit dans le réacteur à raison de 100 moles par atome de W présent. A des intervalles de temps déterminés on analyse la phase gazeuse pour connaître la proportion et la stéréochimie du butène-2 formé.

Le tableau I indique les % et les rapports trans/cis du butène-2 formé après des temps variables pour chacun des catalyseurs essayés.

**TABLEAU I**

Rendements mol. % en butène-2 et rapports trans/cis (t/c) de butène-2, pour les 5 catalyseurs:

| Exemple n° | 2 | | 3 | | 4 | | 5 | | 6 | |
|---|---|---|---|---|---|---|---|---|---|---|
| Catalyseur | "D" | | "E" | | "J" | | "K" | | $WCl_6 + SnMe_4$ | |
| R dans formule 2 | fluor | | méthyle | | phényle | | chlore | | | |
| Après un temps, en minutes, de: | % | t/c | % | t/c | % | t/c | % | t/c | % | t/c |
| 1 | 4 | 0,9 | - | - | - | - | 3,6 | 0,92 | 0,5 | 0,65 |
| 3 | 15 | 1,1 | 1,5 | 0,19 | 0,8 | 0,2 | 9,9 | 1,07 | 2,5 | 0,70 |
| 6 | 20 | 1,5 | 3 | 0,20 | - | - | 17,5 | 1,42 | 6,6 | 0,75 |
| 10 | 24 | 2,2 | 6 | 0,21 | 14,4 | 0,2 | - | - | 14,6 | 1,00 |
| 20 | 25 | 2,4 | 10 | 0,22 | 21 | 0,39 | 22 | 2,32 | 17 | 1,1 |
| 55 | - | - | 14 | 0,25 | 25 | 0,92 | 25 | 2,35 | 25 | 2,0 |
| 200 | - | - | 17 | 0,30 | 25 | 2,5 | 25 | 2,60 | 25 | 2,5 |

On peut constater que le catalyseur D suivant l'invention permet d'atteindre l'équilibre de métathèse, soit 25 % de conversion, en 20 mn, alors qu'il faut 55 mn pour arriver au même point avec le catalyseur classique de l'exemple 6. D'autre part, il est possible de choisir, parmi les catalyseurs suivant l'invention, ceux qui évitent l'isomérisation de l'oléfine cis en trans, ou - au contraire - ceux qui favorisent cette transformation. Ainsi, en utilisant le catalyseur J (ex. 4), et en arrêtant la métathèse au taux de conversion de 14,4 %, on a une isomérisation qui donne seulement 0,2 de forme trans pour 1 de cis, alors qu'il y a un rapport de 1 pour 1 avec le catalyseur classique (ex. 6). De même, peut-on limiter le rapport trans/cis à environ 0,25 par l'utilisation du catalyseur E (ex.3).

Réciproquement, si l'on desire-un fort accroissement de l'isomère trans, on peut obtenir un rapport trans/cis de 2,4 avec le catalyseur D en 20 mn, ou avec K en 55 mn, la conversion étant de 25 % dans les deux cas; il faudrait par contre, pour cela, environ 200 mn avec le catalyseur classique (ex. 6).

<u>EXEMPLES 7 à 9</u>

Il s'agit de la métathèse du pentène-2 cis, effectué dans les mêmes conditions qu'aux exemples 2 à 6, avec des catalyseurs homologues de ceux qui sont désignés plus haut par D, J et K, mais préparés, suivant l'exemple 1, dans de l'éther diisopropylique au lieu de l'éther di-éthylique. Les formules des complexes employés sont donc analogues à celle du composé J (voir exemple 1), la mole d'éther $C_2H_5$-O-$C_2H_5$ étant remplacée par

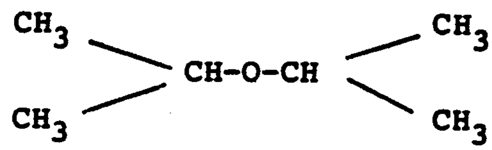

Autrement dit, $R^3$ de la formule 3 est un isopropyle. Dans ces complexes, les R des formules 2 et 3 sont:
des phényles pour le catalyseur F,
des atomes de Cl pour le catalyseur G et
de atomes de F pour le catalyseur H

Il s'en suit que la seule différence entre ces catalyseurs et ceux des exemples précédents réside dans leur

combinaison avec 1 mole de $(CH_3)_2CH$-O-$CH(C_3)_2$ au lieu de $C_2H_5O$-$C_2H_5$. Ainsi :

"F" est l'analogue de "J" (R = phényle)
"G" est l'analouge de "K" (R = chlore)
"H" est l'analogue de "D" (R = fluor)

Le tableau II, rapporte les résultats de métathèse obtenus.

**TABLEAU II**

Rendements mol. % en butène-2 et rapports trans/cis (t/c) de butène-2

| Exemple n° | 7 | | 8 | | 9 | |
|---|---|---|---|---|---|---|
| Catalyseur | F | | G | | H | |
| R dans formule 2 | phényle | | chlore | | fluor | |
| Après un temps, en minutes, de : | % | t/c | % | t/c | % | t/c |
| 1 | 12 | 0,4 | 1 | 0,8 | 0,5 | 0,8 |
| 3 | 22 | 1,0 | 3 | 0,8 | 1,5 | - |
| 6 | 25 | 1,7 | 6,5 | 0,82 | 3,0 | - |
| 10 | 25 | 1,9 | 12 | 0,95 | 4,5 | - |
| 20 | - | | 21 | 1,4 | 6,0 | 0,8 |
| 55 | - | | 25 | 2,2 | 7,0 | - |
| 200 | - | | 25 | 2,5 | 9,0 | 0,9 |

Le catalyseur F (ex. 7) est remarquable en ce qu'il permet d'arriver à l'équilibre de métathèse en 6 minutes; il fait d'ailleurs évoluer peu le rapport trans/cis jusqu'à environ 20 % de conversion qui se situe à moins de 3 minutes.

Le catalyseur G peut être intéressant au point de vue stéréochimique, puisqu'il fait peu varier le taux en isomère trans jusqu'à environ 12 % de conversion (10 minutes). La variation du rapport t/c est également lente avec le catalyseur H: entre la 1ère et la 200ème minute t/c ne change que de 0,8 à 0,9.

La comparaison entre respectivement exemples 7 et 4 (F et J) , 8 et 5 (G et K) ou 9 et 2 (H et D) indique que le changement de l'éther $R^3$-O-$R^3$ dans le complexe a modifié le comportement du catalyseur dans le sens de l'élargissement des possibilités offertes par l'invention.

EXEMPLES 10 à 15
Métathèse du pentène-2 trans

Dans ces exemples sont comparées les activités et les stéréosélectivités des catalyseurs J, K, D, E, G décrits plus haut avec celles du système catalytique, classique, de métathèse, $W(CO)_5PPh_3.ETAlCl_2 + O_2$, dont la mise en oeuvre est décrite, par exemple, dans la publication M. LECONTE et J.M. BASSET, "Journal of American Chemical Society", 101 (1979) p. 7296 - 7302.

Les essais consistent à effectuer la métathèse de pentène-2-trans suivant la réaction

$$2\ CH_3CH=CHCH_2CH_3 \rightleftharpoons CH_3CH=CHCH_3 + CH_3CH_2CH=CHCH_2CH_3$$

donnant du butène-2 et de l'hexène-3.

Les conditions opératoires sont les mêmes que dans les exemples 2 à 6, sauf que la quantité de chacun des catalyseurs K, D, E et G est de 20 x $10^{-6}$ mole, celle de J 38 x $10^{-6}$ mole et celle de catalyseur de l'art antérieur 100 x $10^{-6}$ mole.

Le tableau III indique les résultats obtenus.

(Voir le tableau III à la page suivante)

**TABLEAU III**

Rendement mol. % en butène-2 et rapports trans/cis (t/c) de butène-2

| Exemple n° | 10 | | 11 | | 12 | | 13 | | 14 | | 15 | |
|---|---|---|---|---|---|---|---|---|---|---|---|---|
| Catalyseur | D | | E | | G* | | J | | K | | $W(CO)_5PPh_3$ + 4 $EtAlCl_2$ + 0,5 $O_2$ | |
| R dans formule 2 | flour | | méthyle | | chlore | | phényle | | chlore | | | |
| Après le temps, en minutes, de : | % | t/c | % | t/c | % | t/c | % | t/c | % | t/c | % | t/c |
| 0,5 | 1 | 5,3 | - | - | - | - | 2 | 9,5 | 2 | 7,2 | 0,5 | 1,25 |
| 2 | 4 | 5,3 | - | - | 1,5 | 8,1 | 12 | 9,5 | 10 | 2,6 | 8 | 1,61 |
| 5 | 10 | 4,3 | 1,5 | 9,6 | 9 | 5,7 | 19 | 9,5 | 25 | 2,4 | 12 | 1,72 |
| 15 | 24 | 2,4 | 3,4 | 9,1 | 24 | 2,4 | 22 | 3,8 | 25 | 2,4 | 16,5 | 1,91 |
| 30 | 25 | 2,4 | 6,0 | 8,5 | 25 | 2,4 | 25 | 3,0 | - | - | 19,5 | 1,95 |
| 60 | - | - | 10 | 7,4 | - | - | 25 | 2,6 | - | - | 22 | 2,00 |
| 180 | - | - | 18 | 5,5 | - | - | - | - | - | - | 25 | 2,40 |

*) Complexe avec l'éther $(CH_3)_2CH\text{-}O\text{-}CH(CH_3)_2$ au lieu de $C_2H_5\text{-}O\text{-}C_2H_5$ dans les autres catalyseurs.

On peut constater que les catalyseurs D, G, J et K sont remarquables au point de vue de l'activité, puisqu'ils permettent d'atteindre l'équilibre de 25 % de conversion en 15 à 30 minutes alors qu'il faut 180 mn avec le catalyseur de l'art antérieur (exemple 15).

D'autre part, tous les catalyseurs (D à K) suivant l'invention sont fort intéressants pour l'obtention de butène-2 riche en forme trans, c'est-à-dire avec un rapport t/c élevé. Il suffit, dans certains cas, de se contenter d'une conversion un peu limitée, pour obtenir du butène à 90 % d'isomère trans. Par contre, le catalyseur connu, à $W(CO)_5PPh_3$, donne toujours un produit contenant plus de 29 % de forme cis.

Ces faits sont illustrés ci-après, au tableau IV, par des données tirées du tableau III. Les teneurs en isomère trans y sont calculées pour différents rendements en butène exprimés en mol. %.

**TABLEAU IV**

| Exemple n° | Catalyseur | Temps en mn | Rendement mol. % | % trans |
|---|---|---|---|---|
| 10 | D | 5' | 10 | 81 |
| 11 | E | 60' | 10 | 88 |
| " | " | 180' | 18 | 84 |
| 12 | G | 5' | 9 | 85 |
| " | " | 15' | 24 | 70 |
| 13 | J | 5' | 19 | 90 |
| " | " | 15' | 22 | 79 |
| 14 | K | 2' | 10 | 72 |
| " | " | 5' | 25 | 70 |
| 15 | $W(CO)_5PPh_3$ | 5' | 12 | 63 |
| " | " | 60" | 22 | 67 |
| " | " | 180 | 25 | 70 |

Ainsi peut-on, avec les catalyseurs, suivant l'invention, avoir une teneur en isomère trans de 70 à 90 % moyennant une durée de métathèse d'environ 5 à 60 minutes, tandis que le catalyseur de la technique antérieure (ex. 15) n'en donne que 60 à 70 %, et pour atteindre 70 % il faut 180 minutes.

Il est également intéressant de noter encore que les propriétés particulières du catalyseur suivant l'invention peuvent être influencées par la nature de l'éther combiné au complexe. On voit, en effet, que K, porteur d'une mole de $C_2H_5OC_2H_5$, est plus intéressant pour la cinétique de la métathèse, mais bien moins en ce qui concerne la stéréosélectivité que G qui renferme une molle d'éther isopropylique $[(CH_3)_2CH]_2O$.

EXEMPLES 16 à 18

Métathèse d'une oléfine porteuse de groupes fonctionnels (oléate d'éthyle cis)

Les essais consistent à maintenir à 80°C, en présence d'un catalyseur à base de W, de l'oléate d'éthyle cis, et à déterminer le % d'octadécène-9 et la stéréochimie de cet octadécène formé suivant la réaction équilibrée:

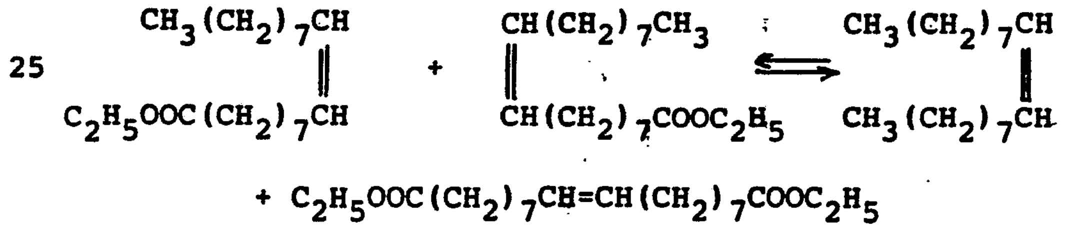

qui fournit, en même temps, de l'hexadécène-8 di-1,16-carboxylate d'éthyle.

La réaction est réalisée dans un appareil pour travail discontinu, purgé préalablement à l'argon, dans lequel on introduit $10^{-4}$ mole de composé tungstique, éventuellement un co-catalyseur en une quantité qui est précisée dans le Tableau V, et 5 ml de chlorobenzène en tant que solvant.

Après le chauffage du réacteur à 80° C, l'oléate d'éthyle cis est introduit en une quantité indiquée au Tableau V sous la forme du nombre de moles d'oléate par atome de W présent.

A certains intervalles de temps, on analyse la phase liquide du réacteur, pour déterminer le rendement en octadécène-9, c'est-à-dire le rapport % du nombre de moles de ce composé formé au nombre de moles d'oléate mis en oeuvre; on détermine également le rapport trans-octadécène-9/cis-octadécène-9. Le système catalytique désigné par "WPb" (exemple 17) est constitué par

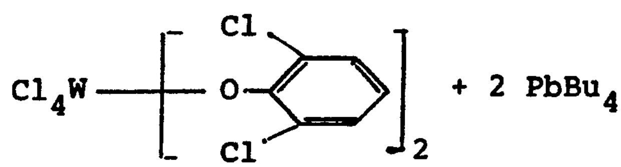

dont il est question dans la demande de brevet FR-8 409 001.

**TABLEAU V**

Rendements mol/% en octadécène-9 et rapports trans/cis octadécène-9 (t/c)

| Exemple n° | 16 | | 17 | | 18 |
|---|---|---|---|---|---|
| Catalyseur | J | | "WPb" | | $WCl_6+2SnMe_4$ |
| Oléate d'éthyle mol / W mol | 125 | | 50 | | 50 |
| Après les temps en mn: | % | t/c | % | t/c | % |
| 10 | 13,5 | 0,55 | 5 | 1,8 | - |
| 30 | 16 | 1,0 | 14 | 2,57 | 4,2 |
| 60 | 16 | 1,42 | 14 | 2,70 | - |
| 120 | - | - | 14 | - | 5,0 |
| 1200 | 16 | 4,0 | 15 | 4,20 | 6,0 |

Ces résultats montrent que le catalyseur de l'invention J est bien plus actif, sans aucun co-catalyseur, que le système catalytique classique (ex. 18) $WCl_6$ + $SnMe_4$; il l'est également plus que le système catalytique de la demande de brevet 8 409 001 (ex. 17). En effet, même avec un rapport oléate/W plus élevé, on obtient avec le catalyseur J un rendement de 13,5 % en octadécène-9 après 10 mn, alors qu'après le même temps ce rendement n'est que de 5 % avec le système catalytique de l'exemple 17.

Le catalyseur J selon l'invention est aussi particulièrement intéressant pour sa stéréosélectivité. En effet au rendement de 13,5 %, proche du rendement maximum qui est de 16 %, on observe que l'octadécène-9 formé est encore en majeure partie cis (64,5 % cis) alors que l'équilibre thermodynamique est nettement en faveur du produit trans. Par contre, avec le catalyseur de l'exemple 17, à seulement 5 % de rendement, le rapport trans/cis est déjà égal à 1,8, c'est-à-dire la proportion de cis n'est plus que 35,7 %.

EXEMPLES 19 à 22

Application de catalyseurs selon l'invention à la métathèse croisée entre des esters d'acides oléfiniques et des oléfines, en vue de la synthèse de phéromones d'insectes.

La réaction étudiée est :

$$3\ CH_3(CH_2)_7CH{=}CH(CH_2)_7COOEt\ (I)\ +\ CH_3(CH_2)_3CH{=}CH(CH_2)_3CH_3\ (II)$$

$$\rightleftharpoons$$

$$EtOOC(CH_2)_7CH{=}CH(CH_2)_7COOEt\ (III)\ +\ CH_3(CH_2)_3CH{=}CH(CH_2)_7COOEt\ (IV)$$

$$+\ CH_3(CH_2)_7CH{=}CH(CH_2)_7CH_3\ (V)\ +\ CH_3(CH_2)_3CH{=}CH(CH_2)_7CH_3\ (VI)$$

Cette réaction, entre l'oléate d'éthyle et le décène-5, a été réalisée dans un appareil de type discontinu, convenablement purgé à l'argon. Les réactifs ont été introduits dans l'ordre suivant:

$0{,}33 \times 10^{-4}$ mole de catalyseur,
5 ml de chlorobenzène (solvant),
$0{,}125 \times 10^{-2}$ mole d'oléate d'éthyle + $0{,}25 \times 10^{-2}$

Le réacteur est chaffé à 85°C et l'analyse de la phase liquide au cours du temps permet de déterminer la conversion en moles % de l'oléate de départ et la sélectivité (moles %) en produit IV; cette sélectivité représente la quantité du produit IV formé par rapport à la quantité d'oléate d'éthyle converti.

Le tableau VI rapporte les résultats obtenus avec les catelyseurs F et J selon l'invention à côté de ceux de l'art antérieur utilisant les systèmes catalytiques.

($WCl_6$ + $SnMe_4$) désigné par "WSn" et

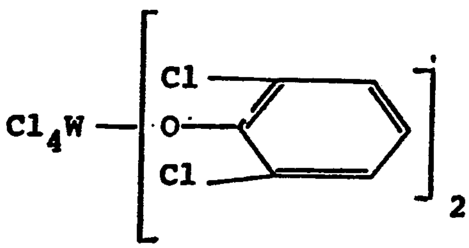

+$PbBu_4$ désigné par "WPb"

**TABLEAU VI**

| EXEMPLES n° | Catalyseur | Rapport molaire (I+II)/W | Temps de réaction | Conversion de I | Sélectivité en IV |
|---|---|---|---|---|---|
| 19 | "WSn" | 50 | 1h | 50 % | 35 % |
| | | | 5h | 60 % | 40 % |
| 20 | "WPb" | 112 | 1h | 72 % | 66 % |
| | | | 2h | 72 % | 66 % |
| 21 | J | 112 | 1/2h | 60 % | 90 % |
| | | | 2h30 | 80 % | 90 % |
| | | | 4h30 | 84 % | 90 % |
| 22 | F | 150 | 1/4h | 66 % | 88 % |
| | | | 1/2h | 80 % | 88 % |
| | | | 1h | 86 % | 88 % |

On peut constater qu'en l'absence de tout co-catalyseur, les complexes F et J selon l'invention permettent de réaliser la réaction de métathèse croisée entre l'oléate d'éthyle et le décène-5 avec des vitesses plus grandes et de bien meilleures sélectivités que les systèmes catalytiques de l'art antérieur, y compris celui qui, Pourtant, était déjà amélioré ("WPb" - exemple 20). En effet, si l'on trace la courbe de la conversion en fonction du temps, à partir des données du tableau VI, on trouve les pentes suivantes en % par heure, pendant la première heure:

| | | |
|---|---|---|
| n°19"WSn" | | 50 |
| 20"WPb" | | 72 |
| 21 | J | 100 |
| 22 | F | 145 |

En outre, avec les catalyseurs connus, on ne dépasse pas 72 % de conversion, alors que celle-ci atteint 86 % avec les catalyseurs de l'invention. En ce qui concerne les sélectivités, elles se situent entre 88 et 90 % contre 40 à 66 % pour l'art antérieur.

EXEMPLE 23

Polymérisation par métathèse d'une oléfine cyclique

La polymérisation par métathèse du dicyclopentadiène est effectuée à l'aide des catalyseurs (K), (D), (F) et (G) de l'invention, chacun utilisé dansune opération discontinue.

Dans un appareil purgé à l'argon, on procède sur les quantités suivantes de réactifs introduits dans l'ordre:

0,5 x $10^{-4}$ mole de catalyseur (K), (D), (F) ou (G)

5 ml de chlorobenzène comme solvant,

3,4 ml soit 2,5 x $10^{-2}$ mole de dicyclopentadiène.

La réaction est poursuivie à 25°C pendant 10 minutes. On trouve alors un rendement de 100 % en polymère, dans chacun des quatre essais.

Les polymères, obtenus par le procédé de l'invention, peuvent être mis en forme par tout moyen connu et en particulier par la pull-extrusion.

**Revendications**

1. Procédé perfectionné de métathèse d'oléfines par contact d'une ou de plusieurs oléfines avec un catalyseur comprenant un complexe de tungstène avec deux groupes phénoxy, porteurs de substituants, et quatre autres ligandes, le complexe portant, comme groupes de coordination, un atome d'halogène, un radical alkyle et un carbène, caractérisé en ce que le catalyseur complexe répond à la formule

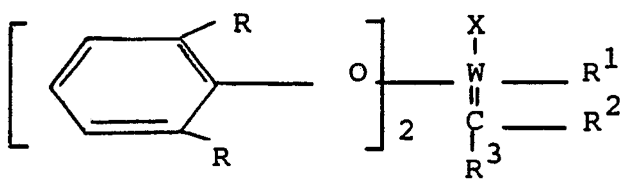 (1)

où X est un halogène, R un groupe hydrocarboné ou un groupe ou atome électronégatif, $R^1$ un groupe alkyle, de préférence en $C_1$ à $C_8$, linéaire ou ramifié et $R^2$ et $R^3$ des atomes d' hydrogène ou des groupes alkyles linéaires ou ramifiés en $C_1$ à $C_8$; $R^1$, $R^2$ et $R^3$ peuvent être semblables ou différents.

2. Procédé suivant la revendication 1, dans lequel R est un alkyle en $C_1$ à $C_6$, un phényle ou un atome de Cl, Br ou F.

3. Procédé suivant la revendication 1 ou 2, caractérisé en ce qu'une ou plusieurs molécules d'un éther $R^4$-O-$R^4$ sont combinées au complexe employé, $R^4$ étant un alkyle en $C_2$ à $C_6$.

4. Procédé suivant la revendication 3, caractérisé en ce que l'éther est de la forme 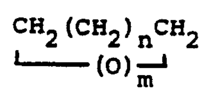

où n est 0 à 6 et m = 1 ou 2.

5. Procédé suivant une des revéndications 1 à 3, caractérisé en ce que le complexe est employé sans aucun co-catalyseur, entre 0°C et 100°C, de préférence 50° à 95°C, la proportion d'oléfine étant de 30 à 300 moles, de préférence 50 à 150 moles, par atomes de W présent.

6. Procédé suivant une des revendications 1 à 3, caractérisé en ce que le complexe est employé avec un co-catalyseur acide de Lewis, tel que composés organo métalliques à base d'Al, Sn, Pb, Mg ou Ti.

7. Catalyseur pour la réalisation de la métathèse suivant une des revendications 1 à 5, comprenant un complexe diphénoxylique du tungstène, caractérisé par la structure:

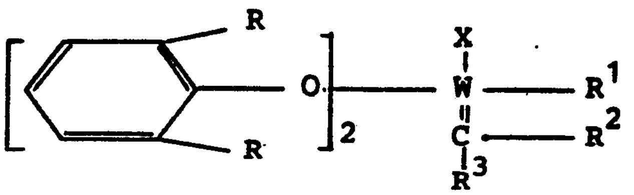

(1)

où X est un halogène, R un groupe hydrocarboné ou un groupe ou atome électronégatif, $R^1$ un groupe alkyle, de préférence en $C_1$ à $C_8$, linéaire ou ramifié et $R^2$ et $R^3$ des atomes d'hydrogène ou des groupes alkyles linéaires ou ramifiés en $C_1$ à $C_8$; $R^1$, $R^2$ et $R^3$ peuvent être semblables ou différents.

8. Catalyseur suivant la revendication 7, caractérisé en ce qu'une mole d'éther du type $R^4OR^4$ ou

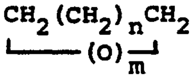

est combinée à la molécule du complexe, $R^4$ étant un alkyle en $C_2$ à $C_6$, n 0 à 6 et m = 1 ou 2.

9. Catalyseur suivant la revendication 7, caractérisé en ce que R est un phényle, un méthyle, un atome de Cl ou de F, $R^1$ un néopentyle, $R^2$ un tert.-butyle et $R^3$ un atome d'hydrogène.

10. Catalyseur suivant la revendication 8, caractérisé en ce que $R^4$ est un éthyle ou un isopropyle.

11. Procédé de préparation d'un catalyseur complexe suivant une des revendications 7 à 10, caractérisé en ce que l'on fait réagir un magnésien porteur des groupes $R^1$ sur un complexe tétrahalogéné du tungstène diphénoxylé

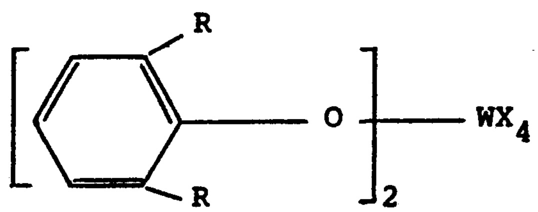

au sein d'un éther.

12. Application du catalyseur suivant une des revendications 7 à 10 à la métathèse d'oléfines portant des groupes fonctionnels ou ne portant pas de tels groupes, en particulier en vue de l'obtention de phéromones d'insectes ou/et pour synthétiser des produits enrichis en le stéréoisomère cis ou trans.

13. Application du procédé suivant une des revendications 1 à 6 à la polymérisation d'une oléfine cyclique, en particulier celle du dicyclopentadiène.

**Patentansprüche**

1. Verbessertes Verfahren zur Metathese von Olefinen durch Kontakt eines oder mehrerer Olefine mit einem Katalysator, umfassend einen Wolframkomplex mit zwei Substituenten tragenden Phenoxygruppen und vier anderen Liganden, wobei der Komplex als Koordinationsgruppen ein Halogenatom, einen Alkylrestund ein Carben trägt, dadurch gekennzeichnet, dass der komplexe Katalysator der Formel

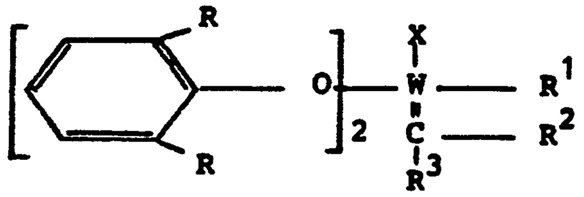

(1)

entspricht, worin X ein Halogen bedeutet, R eine Kohlenwasserstoffgruppe oder eine elektronegative Gruppe oder Atom bedeutet, $R^1$ einen linearen oder verzweigten Alkylrest vorzugsweise mit 1 bis 8 C-Atomen, bedeutet und $R^2$ und $R^3$ Wasserstoffatome oder lineare oder verzweigte $C_1$-$C_8$-Alkylreste bedeuten, wobei $R^1$, $R^2$ und $R^3$ gleich oder verschieden sein können.

2. Verfahren nach Anspruch 1, bei dem R ein $C_1$-$C_6$-Alkylen, ein Phenyl oder ein Atom von Cl, Br oder F ist.

3. Verfahren nach Anspruch 1 oder 2 dadurch gekennzeichnet, dass eines oder mehrere Moleküle eines Ethers $R^4$-O-$R^4$ mit dem Verwendeten Komplex kombiniert sind, wobei R einen $C_2$-$C_6$-Alkylrest bedeutet.

4. Verfahren nach Anspruch 3, dadurch gekennzeichnet, dass der Ether die Form 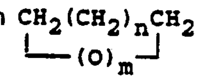 aufweist, wobei n 0 bis 6 und m 1 oder 2 ist.

5. Verfahren nach einem der Ansprüche 1 bis 3, dadurch gekennzeichnet, dass der Komplex ohne Cokatalysator zwischen 0 und 100°C, vorzugsweise 50 bis 95°C, eingesetzt wird, wobei der Olefinanteil 30 bis 300 Mol, vorzugsweise 50 bis 150 Mol, pro vorhandenen W-Atomen beträgt.

6. Verfahren nach einem der Ansprüche 1 bis 3, dadurch gekennzeichnet, dass der Komplex mit einem Lewis-Säure-Cokatalysator eingesetzt wird, wie metallorganische Verbindungen auf der Basis von Al, Sn, Pb, Mg oder Ti.

7. Katalysator zur Durchführung der Metathese nach einem der Ansprüche 1 bis 5, umfassend einen Diphenolkomplex von Wolfram, der durch folgende Struktur gekennzeichnet ist:

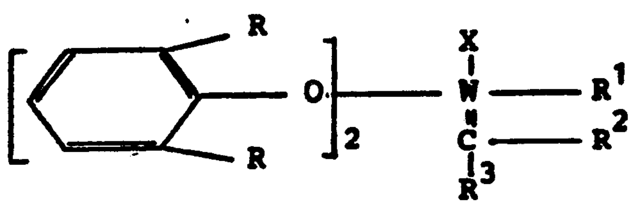

(1)

worin X ein Halogen bedeutet, R eine Kohlenwasserstoffgruppe oder eine elektronegative Gruppe oder Atom bedeutet, $R^1$ einen linearen oder verzweigten Alkylrest, vorzugsweise mit 1 bis 8 C-Atomen bedeutet, und $R^2$ und $R^3$ Wasserstoffatome oder lineare oder verzweigte $C_1$-$C_8$-Alkylreste bedeuten, wobei $R^1$, $R^2$ und $R^3$ gleich oder verschieden sein können.

8. Katalysator nach Anspruch 7, dadurch gekennzeichnet, dass 1 Mol Ether vom Typ $R^4OR^4$ oder

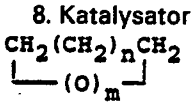

mit dem Komplexmolekülkombiniert ist, wobei $R^4$ einen $C_2$-$C_6$-Alkylrest bedeutet, n 0 bis 6 und m 1 oder 2 ist.

9. Katalysator nach Anspruch 7, dadurch gekennzeichnet, dass R Phenyl, Methyl, ein Cl oder F-Atom, $R^1$ Neopentyl, $R^2$ tert.-Butyl und $R^3$ ein Wasserstoffatom bedeutet.

10. Katalysator nach Anspruch 7 und 8, dadurch gekennzeichnet, dass $R^4$ Ethyl oder Isopropyl bedeutet.

11. Verfahren zur Herstellung eines komplexen Katalysators nach einem der Ansprüche 7 bis 10, dadurch gekennzeichnet, dass man eine $R^1$-Gruppen tragende Magnesiumverbindung auf einen Tetrahalogenkomplex von Diphenoxylwolfram

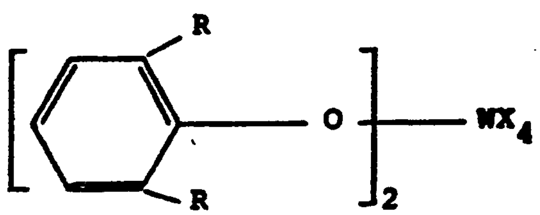

in einem Ether einwirken lässt.

12. Anwendung des Katalysators nach einem der Ansprüche 7 bis 10 zur Metathese von Olefinen, die funktionelle Gruppen tragen oder keine derartigen Gruppen tragen, insbesondere zur Herstellung von Insektenpheromonen und/oder zur Synthese ovon Produkten, die in Bezug auf das cis- oder trans-Stereoisomere angereichert sind.

13. Anwendung des Verfahrens nach einem der Ansprüche 1 bis 6 zur Polymerisation eines cyclischen Olefins, insbesondere von Dicyclopentadien.

**Claims**

1. Improved process of the metathesis of olefins by contact of one or more olefins with a catalyst comprising a tungsten complex with two phenoxy groups carrying substituents and four other ligands, the complex carrying as coordination groups a halogen atom, an alkyl radical and a carbene, characterised in that the complex catalyst corresponds to the formula

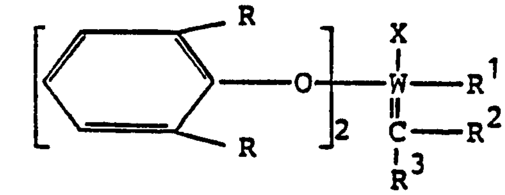

(1)

where X is a halogen, R is a hydrocarbon group or an electro-negative group or atom, $R^1$ is a straight or branched alkyl group, preferably $C_1$ to $C_8$, and $R^2$ and $R^3$ are hydrogen atoms or straight or branched $C_1$ to $C_8$ alkyl groups; $R^1$ , $R^2$ and $R^3$ can be the sane or different.

2. Process according to claim 1, in which R is a $C_1$ to $C_6$ alkyl, a phenyl or a Cl, Br or F atom.

3. Process according to claim 1 or 2, characterised in that one or are molecules of an ether $R^4$-O-$R^4$ are combined with the complex employed, $R^4$ being a $C_2$ to $C_6$ alkyl.

4. Process according to claim 3, characterised in that the ether is in the form 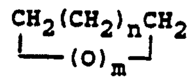

where n is 0 to 6 and m 1 or 2.

5. Process according to any of claims 1 to 3, characterized in that the complex is employed without any co-catalyst between 0° and 100°C, preferably 50° to 95°, the proportion of olefin being from 30 to 300 moles and preferably 50 to 150 moles per atom of W present.

6. Process according to any of claims 1 to 3, characterized in that the complex is employed with a Lewis acid co-catalyst, such as organo-metallic compounds based on Al, Sn, pb, Mg or Ti.

7. Catalyst for carrying out the metathesis according to any or claims 1 to 5, comprising a di-phenoxy tungsten complex, characterised by the structure

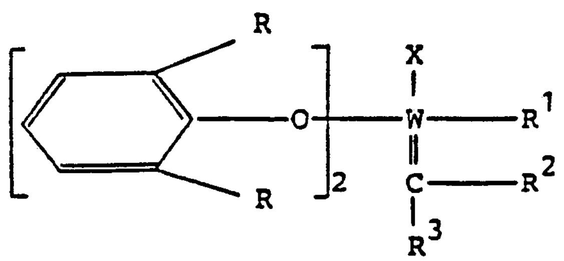

(1)

where X is a halogen, R is a hydrocarbon group or an electro-negative group or atom, $R^1$ is a straight or branched alkyl group, preferably $C_1$ to $C_8$, and $R^2$ and $R^3$ are hydrogen atoms or straight or branched $C_1$ to $C_8$ alkyl groups; $R^1$, $R^2$ and $R^3$ being the same or different.

8. Catalyst according to claim 7, characterised in that a mole of ether of the $R^4OR^4$ or 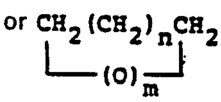

is combined with the molecule of the complex, $R^4$ being a $C_2$ to $C_6$ alkyl, n being 0 to 6 and m = 1 or 2.

9. Catalyst according to claim 7, characterised in that R is a phenyl, a methyl, a Cl or F atom, $R^1$ is a neopentyl, $R^2$ is a tert.butyl and $R^3$ is a hydrogen atom.

10. Catalyst according to claim, caracterised in that $R^4$ is an ethyl or an isopropyl.

11. Process of preparation of a complex catalyst according to any of claims 7 to 10, characterised in that a magnesium carrying $R^1$ groups is reacted with a tetrahalogeno complex of di-phenoxy tungsten

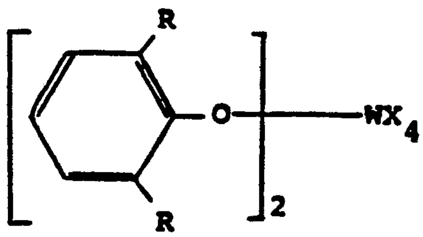

in an ether.

12. Use of the catalyst according to any of claims 7 to 10, for the metathesis of olefins carrying functional groups or not carrying such groups, in particular with a view to obtaining insect pheromones and/or for synthesising products enriched in the cis or trans stereoisomer.

13. Use of the process according to any of claims 1 to 6, for the polymerization of a cyclic olefin, in particular dicyclopentadiene.